# EUROPEAN PATENT SPECIFICATION

(11) **EP 3 937 862 B1**
(45) Date of publication and mention of the grant of the patent: **30.08.2023**
(21) Application number: 20715689.4
(22) Date of filing: 11.03.2020
(51) Int. Cl.: A61F 5/443, A61F 5/445, A61F 5/44, A61F 5/441

(54) **AN OSTOMY WAFER CONSTRUCTION**
AUFBAU EINER OSTOMIESCHEIBE
CONSTRUCTION D'UNE PLAQUETTE DE STOMIE

(30) Priority: 11.03.2019 DK PA201900307; 05.09.2019 DK PA201901049
(43) Date of publication of application: 19.01.2022
(73) Proprietor: Furine ApS, 3320 Skævinge (DK)
(72) Inventor: NIELSEN, Brian Thomsen, 3330 Gørløse (DK)
(74) Representative: Guardian IP Consulting I/S
(86) International application number: PCT/EP2020/056577
(87) International publication number: WO 2020/182923

(56) References cited:
- EP-A1- 2 654 633
- WO-A1-2005/102229
- GB-A- 1 550 960
- US-A1- 2007 219 514
- US-A1- 2012 041 404
- US-A1- 2012 302 981
- US-A1- 2012 323 193

## Description

### Field of the invention

The present specification discloses a first invention related to a wafer construction and a collecting system for application on the peri-stomal skin. The first invention furthermore relates to a method for providing such a construction and/or system.

### Description of related art for the first invention

Many ostomy applications are disclosed in the prior art. Some are arranged as 1-piece collecting bags attached directly to the peri-stomal skin and some are arranged as 2-piece systems with a wafer attached directly to the peri-stomal skin and with a coupling for detachably attaching a bag in such a manner that the bag may be changed more frequently than the wafer. Most systems are attached via adhesive means.

It is known, that output from the ostomy may migrate under the adhesive causing the adhesive to separate from the skin. Problems with such separation are leakages and skin irritation (stomal output may be extremely potent in skin degradation). This is often solved in the prior art by use of liquid absorbing adhesives such as Hydrocolloid. This increases the time before the applied adhesive separates from the skin and reduces the wetting of the skin. However the problem remains.

When inventing the skin attaching portion of an ostomy application, some have tried to mix adhesives and thereby obtain zones with different adhesive properties. Such properties include absorption, adhesive power, flexibility and permeability of the wafer. A wafer construction with zones having different properties may also be achieved by different supporting layers attached to the skin contacting adhesive.

Examples of such ostomy applications comprising an at least 2-zoned adhesive wafer construction with different properties include EP2654633B1, US2014323941A, US2014114265A, US2013274696A and WO 05102229A1.

EP2654633B1 describes a wafer construction for attachment to the peri-stomal skin and being capable of attaching a collecting bag to its outer surface. The wafer comprises a central zone, an intermediate zone and an edge zone. The central zone and edge zone comprise a pressure sensitive adhesive with high adhesive power while the central zone comprises a soft adhesive with lower adhesion power. The soft adhesive has high moist wapor transmission rate (p. 4, line 14-16). The wafer may comprise an impervious backing layer. Furthermore, the wafer may comprise an absorbent layer behind the soft adhesive (p. 4, line 29-32 and figures).

WO2012 083964A1 (found as closest prior art) describes a wafer construction for attachment to the peri-stomal skin. The wafer comprises a central zone and an edge zone. The edge zone provides the majority of the adhesive power and may be moist vapour permeable. The central zone comprises a cushioning layer and may be moist vapour impermeable. The cushioning layer may be a hydrocolloid adhesive layer. The construction comprises a collecting bag to be attached to the wafer either directly (one piece system) via a flange system for detachably attaching the bag to the wafer if designing a two piece system (section 10; p.4 line 26 to p.5 line 2).

Most ostomy collecting systems comprise a skin-attaching wafer, said wafer comprising a skin adhesive on the inside. When analysing the in-use-situation of ostomy bag systems and when viewing the radially extending skin attaching portion from the ostomy, there are two areas. A first central stomal-adjacent-area where the outside of the central portion of the skin-attaching wafer is in fluid communication with the interior of the collecting bag. Also a second encircling area radially more distant from the ostomy, where the outside of the skin-attaching wafer is not in fluid communication with the interior of the collecting bag.

In the interior of the collecting bag, there is mal-odour and 100 % humidity. Even though the prior art may solve flexibility, absorption of liquid and to a certain extent moisture evaporation; when focussing on moisture evaporation from the peri-stomal skin, vapour transportation and water condensation the prior art suffers from a significant drawback. They bring either:
a) via a permeable layer the central stomal-adjacent-area of the skin in vapour communication with the interior of the bag; or
b) they arrange the central stomal-adjacent-area of the wafer to be substantially moisture impermeable.

EP2654633B1 is only capable of handling a certain amount of liquid due to the encapsulated absorbent layer and the bag construction and does not sufficiently handle the combination of strong adherence, skin protection and high skin evaporation. Particularly, the skin in the central stomal-adjacent-area will not be able to expose moisture, when the absorbent layer is saturated.

WO2012 083964A1 is only capable of handling moist vapour evaporation from the portion of the skin extending radially outward from the collecting bag or coupling arrangement.

There remains a need for a wafer construction and ostomy device, which allows skin respiration in the central stomal-adjacent-area in such a manner that the skin adhesive remains sufficiently attached to the skin.

The present invention is in particular useful on users that are relatively active or have increased temperature and therefore have increased skin perspiration. The problems mentioned above are solved at least in part with a wafer construction as characterized in claim 1.

### Summary of the first invention

In a first aspect, the present invention relates to a wafer construction (200) for applying a collecting system (100) around an ostomy, the wafer construction comprising:
a. a central through-going aperture (201) for bringing the ostomy (001) in fluid communication with a collecting reservoir (300),
b. an inner radially extending layer (220) for attaching the wafer to the peri-stomal skin (002); said inner layer (220) comprising:
   i. a skin contacting surface (221);
   ii. a moist vapour permeable layer (224);
   iii. an outer surface (226);
c. an outer radially extending layer (230); said outer layer comprising:
   i. a substantially moist vapour impermeable film (232);
   ii. an outer surface (236);
   iii. an inner surface (238);
   iv. a bag element (234) for engaging the wafer (200) to a collecting reservoir (300);
d. a central stoma-adjacent-area (240) comprising a first area of both the inner layer (220) and the outer layer (230); said central stoma-adjacent-area defined radially as the area of the wafer construction (200) closer to the central through-going aperture (201) than the bag element (234) of the outer layer;
e. an encircling area (250) comprising a second area of at least the inner layer (220);
f. at least one radial space (270) in fluid communication with the atmosphere (500); said radial space (270) being located between the outer surface of the inner layer (226) and the inner surface of the outer layer (238); and
g. at least one attachment point (260); said attachment point attaching the outer layer (230) to the inner layer (220);
wherein at least a portion of the radial space (270) is located in the central stoma-adjacent-area (240).

In a second aspect, the present invention relates to a collecting system (100) application on the peri-stomal skin, said system comprising:
a. a wafer construction (200) according to the first aspect of the present invention; and
b. a collecting reservoir (300) for collecting the stomal output.

In a third aspect, the present invention relates to a method of providing a wafer construction (200) or collecting system (100) according to the first or second aspect of the present invention, said method comprising the steps of:
a. providing a wafer construction (200) according to the first aspect of the present invention; and
b. packing the wafer construction.

### Description of related art for the second invention

Body waste collecting systems are known in the art. For example, reference is made to applicant's own disclosures in WO 2018/050856 and WO2019/179586, both of which are incorporated by reference in their entirety. Body waste collecting systems need to be attached to the user's body. Certain systems use an adhesive to attach an attachment member to the user's body.

If the adhesive on the attachment member detaches from the user's body, there is a risk that body fluids and or odours leak from the user. It is desired to reduce this risk as much as possible. Some prior art systems could be said to have a greater risk of detachment than others due to the mechanical arrangement of the attachment member.

### Brief description of the drawings

In the following, the invention will be described in greater detail with reference to embodiments shown by the enclosed figures. It should be emphasized that the embodiments shown are used for example purposes only and should not be used to limit the scope of the invention.
Figures 1a to 1c schematically illustrate different views of an example of a wafer construction (200) and a collecting system (100) according to the present invention.
Figure 2 is a cross sectional view of an example of a wafer construction (200) and a collecting system (100) according to the present invention prior to application.
Figure 3 is a close up of a cross sectional view of the wafer (200) and system (100) from figure 1 when worn by a patient.
Figures 4 to 5 illustrate schematic top views of different patterns of attachment points in examples of wafer constructions (200) and a collecting system (100) according to the present invention.

### Detailed description of the embodiments

The first aspect of the present invention relates to a wafer construction (200) for applying a collecting system (100) around an ostomy, the wafer construction comprising:
a. a central through-going aperture (201) for bringing the ostomy (001) in fluid communication with a collecting reservoir (300),
b. an inner radially extending layer (220) for attaching the wafer to the peri-stomal skin (002); said inner layer (220) comprising:
   a. a skin contacting surface (221);
   b. a moist vapour permeable layer (224);
   c. an outer surface (226);
c. an outer radially extending layer (230); said outer layer comprising:
   a. a substantially moist vapour impermeable film (232);
   b. an outer surface (236);
   c. an inner surface (238);
   d. a bag element (234) for engaging the wafer (200) to a collecting reservoir (300);
d. a central stoma-adjacent-area (240) comprising a first area of both the inner layer (220) and the outer layer (230); said central stoma-adjacent-area defined radially as the area of the wafer construction (200) closer to the central through-going aperture (201) than the bag element (234) of the outer layer;
e. an encircling area (250) comprising a second area of at least the inner layer (220);
f. at least one radial space (270) in fluid communication with the atmosphere (500); said radial space (270) being located between the outer surface of the inner layer (226) and the inner surface of the outer layer (238); and
g. at least one attachment point (260); said attachment point attaching the outer layer (230) to the inner layer (220);
wherein at least a portion of the radial space (270) is located in the central stoma-adjacent-area (240).

In the context of the present invention the term "permeability" shall be understood as Moist Vapour Transmission Rate (MVTR) and are measured according to ASTM test method F1249 (at 38°C, 100% RH). The term "substantially moist vapour impermeable" shall be understood as a MVTR below 400 g/m2/24h. The term "moist vapour permeable" shall be understood as a MVTR of at least 500 g/m2/24h.

In one embodiment of the first aspect of the present invention, the inner radially extending layer (220) has a permeability of at least 600 g/m2/24h. In one embodiment, the inner radially extending layer has permeability of at least 750 g/m2/24h. In one embodiment, the inner radially extending layer has permeability of at least 900 g/m2/24h. Many technologies known in the art can be used to create a permeable layer. These include, but are not limited to breathable films, non-wovens and micro perforated films (when micro perforated, the permeability increases significantly, why micro perforated impermeable films may act as permeable films).

Useful breathable films may be any film with high permeability. Most frequently, polyurethane films are used, but any permeable film may be used including silicone films and membranes.

In one embodiment of the first aspect of the present invention, the moist vapour permeable layer (224) is a breathable film. In one embodiment of the first aspect of the present invention, the moist vapour permeable layer (224) is a silicone film.

In one embodiment of the first aspect of the present invention, the outer radially extending layer (230) has a permeability of 0 to 200 g/m2/24h. In one embodiment the outer radially extending layer has a permeability of 0 to 100 g/m2/24h. In one embodiment the outer radially extending layer has a permeability of 0 to 50 g/m2/24h or 0 to 20 g/m2/24h. In one embodiment of the first aspect of the present invention, the outer radially extending layer (230) comprises a multilayered co-extruded film. Useful impermeable films may be any film with low permeability. An example are Nexcel^{®} MF283, which is a Coextruded five-layer EVA/EVA/PVDC/EVA/EVA film from Sealed Air (MVTR 5-15 g/m2/24h).

The wafer construction should be attached to the peri-stomal skin, either via a belt structure or via adhesive means. When using adhesives, as permeability of the inner layer is important, such adhesives should be applied in a relatively thin layer and should not reduce the permeability too much. Opposite it is also important, that the wafer does not unintentionally drop off.

In one embodiment of the first aspect of the present invention, the inner radially extending layer (220) comprises a skin adhesive (222) for attaching the wafer to the peri-stomal skin. Such skin adhesives could for example be acrylates, polyurethane and silicone adhesives. One example of such adhesives could be DOW CORNING MG-2410 Silicone Adhesive.

To minimize the impact of the adhesive on permeability, one could coat the skin contacting adhesive in a pattern.

In one embodiment of the first aspect of the present invention, the skin adhesive (222) is pattern coated on the inner radially extending layer (220). In one embodiment of the first aspect of the present invention, the skin adhesive (222) is pattern coated covering from 25 to 75% of the area of the inner radially extending layer (220).

As described earlier, one purpose of the present invention is to allow the peri-stomal skin to perspire so skin adhesion remains sufficiently high. This is today not solved by prior art - in particular in the stoma-adjacent-area.

In the present invention, as the inner radially extending layer (220) is not fullwelded onto the outer radial layer (230), there is formed a radial space (270) between the two radially extending layers. This space (270) is in fluid communication with the atmosphere, allowing the perspired moisture to escape from the wafer construction. Consequently, a wafer construction according to the present invention, allows the peri-stomal skin to stay dry (and healthy) opposite to wafer constructions that absorb moisture, for example via hydrocolloid adhesives. On these prior art wafers, even though they do absorb the moisture, the skin tends to be wetted by the absorbed moisture, which again damaged the skin and may lead to wounding and wound infection.

In the present invention, it may be an advantage to somehow facilitate the separation of the inner radially extending layer from the outer radially extending layer, by a separation means, such as foams and non-wovens.

In one embodiment of the first aspect of the present invention, the at least one radial space (270) comprises separations means (271); said separation means facilitating the separation of the inner radially extending layer (220) from the outer radially extending layer (230) in the radial space (270).

In one embodiment of the first aspect of the present invention, the separations means (271) is a non-woven layer. In one embodiment of the first aspect of the present invention, the separations means is an open cell foam structure.

In one embodiment of the first aspect of the present invention, the separations means (271) is attached to the inner surface (238) of the outer layer (230). In one embodiment of the first aspect of the present invention, the separations means is attached to the outer surface (226) of the inner layer (220).

In one embodiment of the first aspect of the present invention, the separations means (271) comprises moisture-transporting capabilities.

In order for the wafer to be integral, the outer layer must be attached to the inner layer - at least when the system is fully applied to the peri-stomal skin. If not, the outer layer (and the collecting bag would drop off). Therefore, the wafer comprises attachment point(s) (260), attaching the outer and inner layers to each other, at least during use. These attachment point(s) (260) may be separate discrete points or one large structure or pattern.

In one embodiment of the first aspect of the present invention, the wafer construction comprises at least one attachment point (260) for attaching the inner layer (220) to the outer layer (230). The attachment point(s) may be created via glue, adhesive, welding or the like. In one embodiment of the first aspect of the present invention, the wafer construction comprises at least two attachment points (260) for attaching the inner layer (220) to the outer layer (230). In one embodiment of the first aspect of the present invention, the attachment point(s) is located both in the central stoma-adjacent-area (240) and in the encircling area (250).

In one embodiment of the first aspect of the present invention, the substantially moist vapour impermeable film (232) of the outer radially extending layer (230) is solely located in the central stoma-adjacent-area (240).

If the radial space (270) comprises separation means (271), in principle the separation means could be attached to both the outer layer (230) and the inner layer (220). In such an example, the outer and inner layers could be attached to each other via one large attachment point.

In one embodiment of the first aspect of the present invention, the separations means (271) is attached to the inner surface (238) of the outer layer (230) and to the outer surface (226) of the inner layer (220).

The portion of the wafer construction intended to be right next to and in some cases contact the ostomy is the inner part of the central stoma-adjacent-area (240). This portion is called the stoma-contacting-portion (245). The stoma-contacting-portion may, depending on the level of contact to the stoma and the sensitivity of the ostomy, be soft and gentle towards the stoma. Such softness may be made via a gel-matrix or via some mouldable wax. The stoma-contacting-portion should not be sharp as it may damage the ostomy.

In one embodiment of the first aspect of the present invention, the central stoma-adjacent-area (240) comprises a stoma-contacting-portion (245), said stoma-contacting-portion being adapted to contact the ostomy. In one embodiment of the first aspect of the present invention, the stoma-contacting-portion (245) comprises a gel. In one embodiment of the first aspect of the present invention, the stoma-contacting-portion (245) comprises an adhesive gel. In one embodiment of the first aspect of the present invention, the gel in the stoma-contacting-portion (245) is of a shore A hardness of less than 16, less than 12 or less than 8. In one embodiment of the first aspect of the present invention, the thickness of the wafer in the stoma-contacting-portion (245) is at least 1 mm, at least 1.5 mm or at least 2 mm.

The second aspect of the present invention relates to a collecting system (100) application on the peri-stomal skin, said system comprising:
a. a wafer construction (200) according to the first aspect of the present invention; and
b. a collecting reservoir (300) for collecting the stomal output.

The collecting reservoir (300) in the second aspect of the present invention may be integrally formed with the wafer (200) similar to typical 1-piece systems or it may be detachably attached to the wafer as known from 2-piece systems.

When the system is integrally formed, the bag element (234) for engaging the wafer (200) to the collecting reservoir (300) is most radially outer positioned where the bag is attached to the wafer for example via welding, glue or adhesion.

When the collecting reservoir (300) is attached to the wafer (200), the bag element (234) is the coupling or adhesive arrangement for detachably attaching the collecting reservoir (300) to the wafer (200).

In one embodiment of the second aspect of the present invention, the collecting system (100) comprises a skin adhesive (400) for supporting the attachment of the system towards the skin.

The skin adhesive is intended to be positioned between the skin contacting surface (221) of the wafer and the peri-stoma skin, supporting the adhesion of the system (100) to the peri-stomal skin. The skin adhesive (400) may be a liquid solvent based adhesive, an uncured in-situ curable adhesive or a layer of double-sided adhesive.

In one embodiment of the second aspect of the present invention, the skin adhesive (400) comprises a solvent (410) for solubilizing the adhesive. In one embodiment of the second aspect of the present invention, the solvent (410) is an oligo-methyl-di-siloxane chemical. In one embodiment of the second aspect of the present invention, the solvent (410) is hexamethyl-disiloxane.

In one embodiment of the second aspect of the present invention, the skin adhesive (400) is a silicone adhesive. Examples of such silicone skin adhesives include Advabond from Advancis Medical and MG-2410 Silicone Skin Adhesive from Dow Corning.

In one embodiment of the second aspect of the present invention, the collecting system (100) comprises a soluble adhesive (400) for application on the skin prior to application of the wafer.

In the stoma-contacting-portion (245), moist perspiration may be reduced if the inner (220) and outer layer (230) are joint via a ring-shaped attachment point (260a). In one embodiment, the ring-shaped attachment point (260a) is formed as an impermeable ring. Therefore, the stoma-contacting area may have a relative small width. On the other hand, to provide broader cutting opportunities for the user, a relative large width of the stoma-contacting-portion (245) is needed. One way of meeting these opposite directed needs in two-piece systems are to provide the stoma-contacting-portion as a separate member for attachment when being used.

In one embodiment of the second aspect of the present invention, the collecting system (100) comprises a separate gel-ring for sealing the system directly on outer diameter of the ostomy, said separate gel-ring be being attachable to a portion of the outer surface (236) of the outer radially extending layer (230).

The third aspect of the present invention relates to a method of providing a wafer construction (200) or collecting system (100) according to the first or second aspect of the present invention, said method comprising the steps of:
a. providing a wafer construction (200) according to the first aspect of the present invention; and
b. packing the wafer construction.

In one embodiment of the third aspect of the present invention, the method of providing a wafer construction (200) comprises the steps of:
a. providing an inner permeable layer (220);
b. providing an outer impermeable layer (230);
c. attaching the inner permeable layer to the outer layer in at least one attachment point; and
d. applying an adhesive on the skin contacting surface of the inner permeable layer.

In one embodiment of the third aspect of the present invention, the method of providing a collecting system (100) comprises the steps of:
a. providing a collecting system according to the second aspect of the present invention; and
b. providing a skin adhesive for application on the peri-stomal skin.

In one embodiment of the third aspect of the present invention, the skin adhesive is a liquid solubilized adhesive.

It will be appreciated that any combination of features and elements of the above described aspects, inventions and/or embodiments may be combined in any suitable manner.

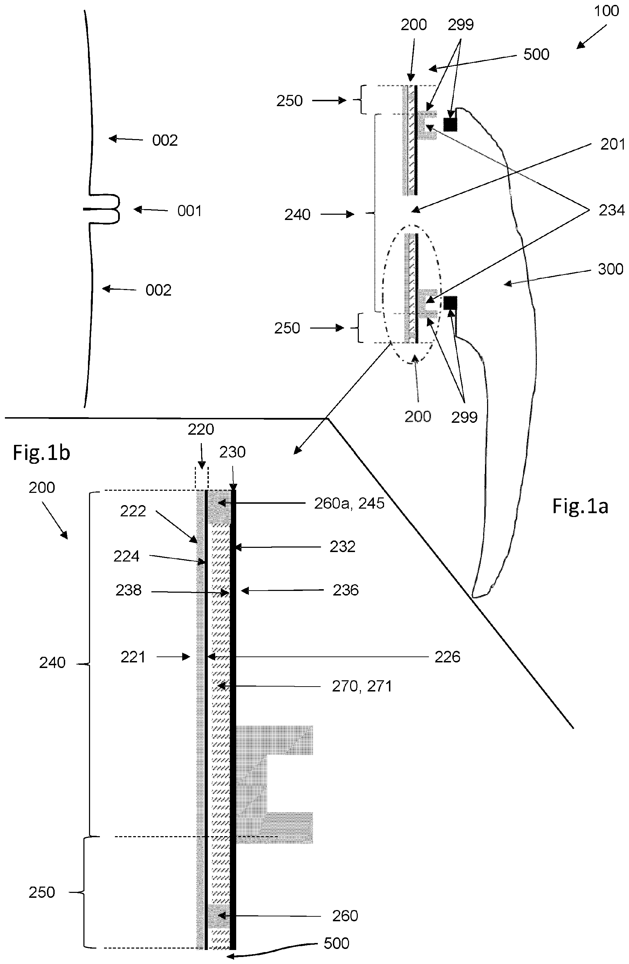

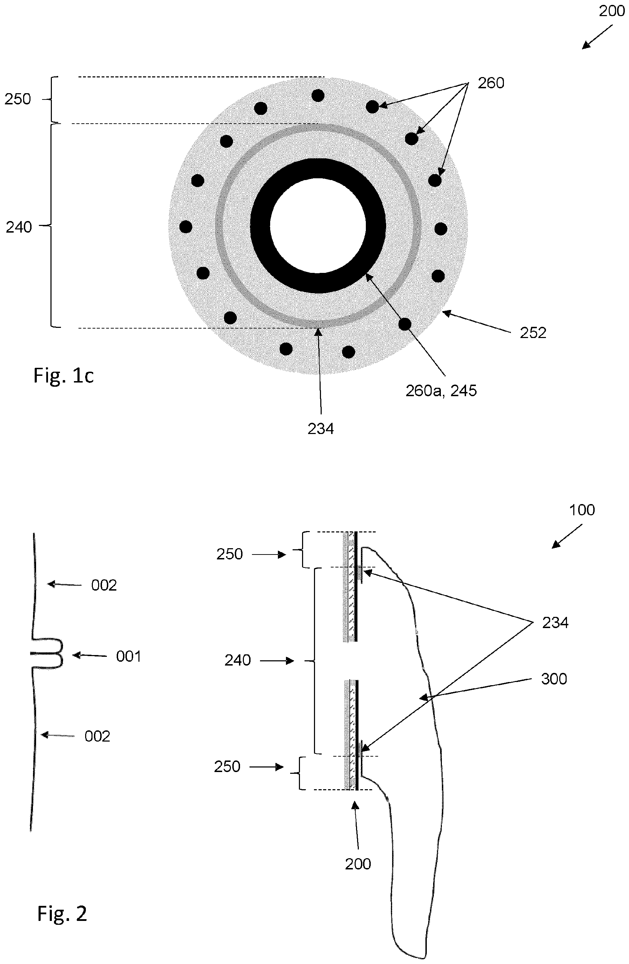

Figure 1 discloses a schematic cross-sectional illustration showing the main components of an example of a collecting system according to the present invention. Figure 1a illustrates the collecting system (100) in close proximity of an ostomy (001) and its peri-stomal skin (002). The system 100 comprises a wafer construction (200) and a collecting reservoir (300), which in this example is an ostomy bag. Figure 1b is a magnified cross-sectional, radial view of the wafer construction (200). Figure 1c is a schematic top view showing the main components of the wafer of this example. The illustrated example is a two-piece system, where the collecting reservoir (300) can be detachably attached to the wafer construction (200) via a coupling arrangement (299) located on both the wafer construction and on the collecting reservoir. Any coupling arrangement can be used, these are known in the prior art and will not be described in the present invention.

The wafer construction comprises a central through-going aperture (201) for receiving the ostomy (001). Furthermore, the wafer construction comprises an inner layer (220) and an outer layer (230), both intended to extend radially along the peri-stomal skin (002). The inner layer (220) comprises in this example a skin adhesive (222) on its skin contacting surface (221). In this example, the skin adhesive (222) is applied directly on a moist vapour permeable layer (224), which is a breathable film. The outer surface of the moist vapour permeable layer (224) constitutes the outer surface (226) of the inner layer (220).

The outer layer (230) of the wafer construction (200) comprises a moist vapour impermeable film (232), which outside surface in this example constitutes the outer surface (236) of the outer layer (230) and of the wafer (200). When the collecting reservoir (300) is attached to the wafer construction (200), the central area of the outer layer (230) of the wafer construction (200) participates in the wall structure of the collecting reservoir (300). In this example, the coupling arrangement (299) on the outer layer is the bag element (234).

The wafer construction (200) is defined to have radially two areas. A first central stoma-adjacent-area (240) and a second encircling area (250). The extent of the central stoma-adjacent-area (240) is defined by the position of the bag element (234) on the outer layer (230), in such a manner, that the central stoma-adjacent-area (240) is radially closer to the central through-going hole (201) than the bag element (234). The remaining portion of the wafer constitutes the encircling area (250).

As the outer and inner layers are not completely laminated or welded directly together, there is formed a radial space (270) between the two layers. The purpose of this space is to allow skin respiration during the use of the wafer and the collecting system. The space (270) must be in fluid communication with the atmosphere to allow optimal respiration. Moist vapour from skin respiration is transported through the permeable inner layer (220) and into the radial space (270). From the radial space (270) moisture is vented into the atmosphere (500).

However, as the inner and outer layer forms one wafer, they may be joined together by attachment point(s) (260). This may be via glue, adhesive, welding or other means for attachning the two layers together. The attachment point(s) (260) should be located in both the central stoma-adjacent-area (240) and in the encircling area (250). In this example, there are multiple attachment points (260) in the encircling area (250) and one ring-shaped attachment point (260a) in the central stoma-adjacent-area (240). The ring shaped attachment point (260a) is in one embodiment made of an impermeable material or welded together via an impermeable welded joint such that odours are not passed through the ring shaped attachment point. The attachment points may be seen on figure 1c (disclosing a schematic top view showing the main components of the wafer from figure 1, seen from the outer surface (236) of the outer layer (230)). The ring-shaped attachment point (260a) is the stoma-contacting-portion (245) of the wafer. In this example, the stoma-contacting-portion is made of a soft silicone gel with a width of 8 mm and a thickness of 2 mm. The stoma-contacting-portion (245) may be cut so the central through-going aperture (201) fits to the shape of the ostomy (001).

The wafer construction (200) may comprise separation means (271), which is disclosed in this example as a non-woven layer. The separation means is located between the inner layer (220) and outer layer (230). The separation means (271) may also act as a moisture transporter, but may also just be positioned to support the separation of the inner layer (220) and the outer layer (230) for improved venting of the space (270) to the atmosphere (500). The spacing layer of this example is disclosed on figure 1b.

Figure 2 illustrates a cross-sectional view of a 1-piece collecting system (100) similar to the 2-piece system illustrated in figure 1. The coupling arrangement (299) from figure 1 is replaced with a welding causing the wafer (200) to be non-detachably attached to the collecting reservoir (300). In this example, the welding is the bag element (234), which radially forms the outer boarder of the stoma-adjacent-area (240). The portion of the outer radially extending layer (230) that is located in the stoma-adjacent-area (240) participates in the formation of the collecting reservoir (300).

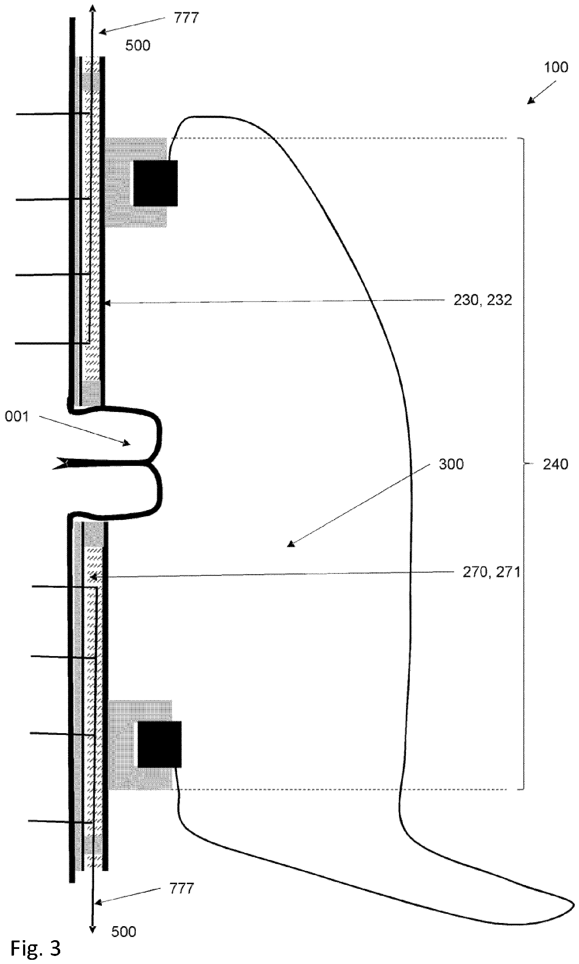

Figure 3 is a cross-sectional close-up of the wafer (200) and system (100) from example 1 applied onto the peri-stomal skin (002). The arrows (777) illustrate how peri-stomal skin perspiration can occur through the inner layer (220) to its outer surface (226) and into the radial space (270). As the radial space is in fluid communication with the atmosphere (500), the perspired moisture can escape the wafer construction and into the atmosphere.

Note, that as opposed to prior art, the peri-stomal skin attached to the central stoma-adjacent-area of the wafer in the present invention is able to expel moisture to the atmosphere. As the outer layer (230) in the central stoma-adjacent-area (240) comprises an impermeable layer (232), mal-odour from the ostomy (001) and from the collecting reservoir (300) is not able to migrate to the atmosphere via the radial space (270). The spacing means (271) in the radial space (270) support the separation of the outer and inner layer and thereby improve the perspiration.

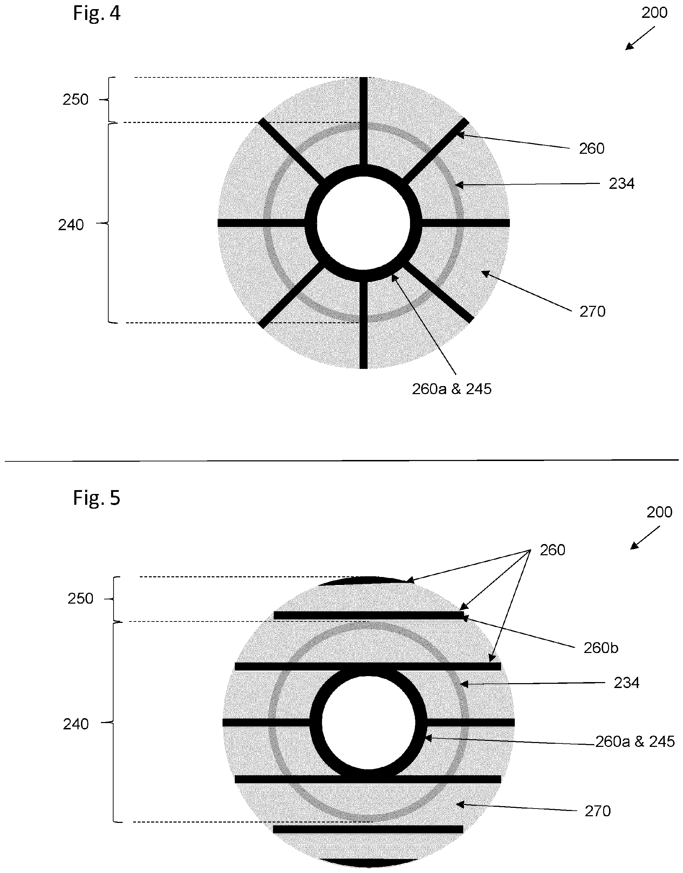

Figure 4 is a schematic top view showing the main components of another example of a wafer according to the first aspect of the present invention. The main difference from figure 1 (figure 4 is directly comparable to figure 1c), is that the attachment point (260) is one large attachment, shaped like a star around the central through-going aperture (201). In this example, the attachment point is a 2.5 mm thick adhesive gel. The central ring shape (260a) of the attachment point (260) is the stoma-contacting-portion (245) of the wafer. The stoma-contacting-portion may be cut to fit the central through-going aperture (201) to the shape of the ostomy (001).

It should be noted, that in this example, due to the star shaped structure of the attachment point (260), there are 8 radial spaces (270).

Figure 5 is a schematic top view showing the main components of another example of a wafer according to the first aspect of the present invention. The main difference from figure 1 and 4 (figure 5 is directly comparable to figure 1c and 4), is that the attachment points (260) are a central stomal-encircling ring (260a) and a plurality of horizontal lines (260b). The central ring shape (260a) of the attachment points (260) is the stoma-contacting-portion (245) of the wafer. It should be noted, that in this example, due to the horizontally lined orientation of the attachment points (260), there are a plurality of horizontally oriented radial spaces (270). This design may on some obese patients better fit to the natural skin folds and thereby increase patient comfort.

In principle, the outer layer (230) only needs to comprise a moist vapour impermeable film (232) in central stoma-adjacent-area (240). Outside - in the encircling area (250) of the wafer (200), the outer layer (230) could be a moist vapour permeable layer, such as a non-woven, a film or not exist at all. However if the outer layer extends throughout the encircling area and there exists attachment points in this area, these support the distribution of force of from the attachment of the collecting reservoir (300) via the wafer (200) to the peri-stomal skin.

## Claims

1. A wafer construction (200) for applying a collecting system (100) around an ostomy, the wafer construction comprising:
a. a central through-going aperture (201) for bringing the ostomy (001) in fluid communication with a collecting reservoir (300),
b. an inner radially extending layer (220) for attaching the wafer to the peri-stomal skin (002); said inner layer (220) comprising:
i. a skin contacting surface (221);
ii. a moist vapour permeable layer (224);
iii. an outer surface (226);
c. an outer radially extending layer (230); said outer layer comprising:
i. a substantially moist vapour impermeable film (232);
ii. an outer surface (236);
iii. an inner surface (238);
iv. a bag element (234) for engaging the wafer (200) to a collecting reservoir (300);
d. a central stoma-adjacent-area (240) comprising a first area of both the inner layer (220) and the outer layer (230); said central stoma-adjacent-area defined radially as the area of the wafer construction (200) closer to the central through-going aperture (201) than the bag element (234) of the outer layer;
e. an encircling area (250) comprising a second area of at least the inner layer (220);
f. at least one radial space (270) in fluid communication with the atmosphere (500); said radial space (270) being located between the outer surface of the inner layer (226) and the inner surface of the outer layer (238); and
g. at least one attachment point (260); said attachment point attaching the outer layer (230) to the inner layer (220);
wherein at least a portion of the radial space (270) is located in the central stoma-adjacent-area (240).

2. A wafer construction (200) according to claim 1, wherein the attaching points (260) are located both in the stoma-adjacent-area (240) and in the encircling area (250).

3. A wafer construction (200) according to claim 1 or 2, wherein the attachment point in the stoma adjacent area is arranged as an impermeable joint between the inner and outer radially extending layers, said impermeable joint extending entirely around an ostomy.

4. A wafer construction (200) according to claim 1, wherein the at least one radial space (270) comprises separations means (271); said separation means facilitating the separation of the inner radially extending layer (220) from the outer radially extending layer (230) in the radial space (270).

5. A wafer construction (200) according to any of the preceding claims, wherein the central stoma-adjacent-area (240) comprises a stoma-contacting-portion (245), said stoma-contacting-portion being adapted to contact the ostomy.

6. A wafer construction (200) according to claim 5, wherein the stoma-contacting-portion (245) comprises an adhesive gel with a shore A hardness of less than 16; and said adhesive gel being at least 1 mm thick.

7. A wafer construction (200) according to any of the preceding claims, wherein the skin contacting surface (221) of the inner layer (220) comprises a skin adhesive.

8. A wafer construction (200) according to any of the preceding claims, wherein the substantially moist vapour impermeable film (232) of the outer radially extending layer (230) is solely located in the central stoma-adjacent-area (240).

9. A collecting system (100) for application on the peri-stomal skin, said system comprising:
a. a wafer construction (200) according to claim 1-8; and
b. a collecting reservoir (300) for collecting the stomal output.

10. A collecting system (100) according to claim 9, wherein the system comprises a soluble adhesive (400) for application on the skin prior to application of the wafer.

11. A method of providing a wafer construction (200) or collecting system (100) according to any of the preceding claims, said method comprising the steps of:
a. providing a wafer construction (200) according to claim 1-8; and
b. packing the wafer construction.

## Patentansprüche

1. Wafer-Konstruktion (200) zum Anlegen eines Sammelsystems (100) um ein Stoma, wobei die Wafer-Konstruktion Folgendes umfasst:
a. eine zentrale durchgehende Öffnung (201), um das Stoma (001) mit einem Sammelbehälter (300) in strömungstechnische Kommunikation zu versetzen,
b. eine sich radial erstreckende innere Schicht (220) zum Anbringen des Wafers an der peristomalen Haut (002); wobei die innere Schicht (220) Folgendes umfasst:
i. eine Hautberührungsoberfläche (221);
ii. eine für feuchten Dampf durchlässige Schicht (224);
iii. eine Außenoberfläche (226);
c. eine sich radial erstreckende äußere Schicht (230); wobei die äußere Schicht Folgendes umfasst:
i. eine im Wesentlichen für feuchten Dampf undurchlässige Folie (232);
ii. eine Außenoberfläche (236);
iii. eine Innenoberfläche (238);
iv. einen Beutelelement (234) zum Ineinandergreifen des Wafers (200) mit einem Sammelbehälter (300);
d. eine zentrale, an das Stoma angrenzende Fläche (240), die eine erste Fläche sowohl der inneren Schicht (220) als auch der äußeren Schicht (230) umfasst; wobei die zentrale, an das Stoma angrenzende Fläche radial als die Fläche der Wafer-Konstruktion (200), die näher an der zentralen durchgehenden Öffnung (201) als das Beutelelement (234) der äußeren Schicht liegt, definiert ist;
e. eine umlaufende Fläche (250), die eine zweite Fläche mindestens der inneren Schicht (220) umfasst;
f. mindestens einen radialen Raum (270) in strömungstechnischer Kommunikation mit der Atmosphäre (500); wobei der radiale Raum (270) zwischen der Außenoberfläche der inneren Schicht (226) und der Innenoberfläche der äußeren Schicht (238) liegt; und
g. mindestens eine Anbringungsstelle (260); wobei die Anbringungsstelle die Außenschicht (230) an der inneren Schicht (220) anbringt;
wobei mindestens ein Abschnitt des radialen Raums (270) in der zentralen, an das Stoma angrenzenden Fläche (240) liegt.

2. Wafer-Konstruktion (200) nach Anspruch 1, wobei die Anbringungsstellen (260) sowohl in der an das Stoma angrenzenden Fläche (240) als auch in der umlaufenden Fläche (250) liegen.

3. Wafer-Konstruktion (200) nach Anspruch 1 oder 2, wobei die Anbringungsstelle in der an das Stoma angrenzenden Fläche als eine undurchlässige Verbindung zwischen der sich radial erstreckenden inneren Schicht und äußeren Schicht eingerichtet ist, wobei sich die undurchlässige Verbindung vollständig um ein Stoma erstreckt.

4. Wafer-Konstruktion (200) nach Anspruch 1, wobei der mindestens eine radiale Raum (270) Trennmittel (271) umfasst; wobei die Trennmittel die Trennung der sich radial erstreckenden inneren Schicht (220) von der sich radial erstreckenden äußeren Schicht (230) in dem radialen Raum (270) erleichtert.

5. Wafer-Konstruktion (200) nach einem der vorstehenden Ansprüche, wobei die zentrale, an das Stoma angrenzende Fläche (240) einen Stomaberührungsabschnitt (245) umfasst, wobei der Stomaberührungsabschnitt dazu angepasst ist, das Stoma zu berühren.

6. Wafer-Konstruktion (200) nach Anspruch 5, wobei der Stomaberührungsabschnitt (245) ein Klebegel mit einer Shore-A-Härte von weniger als 16 umfasst; und das Klebegel mindestens 1 mm dick ist.

7. Wafer-Konstruktion (200) nach einem der vorstehenden Ansprüche, wobei die Hautberührungsoberfläche (221) der inneren Schicht (220) einen Hautkleber umfasst.

8. Wafer-Konstruktion (200) nach einem der vorstehenden Ansprüche, wobei die im Wesentlichen für feuchten Dampf undurchlässige Folie (232) der sich radial erstreckenden äußeren Schicht (230) nur in der zentralen, an das Stoma angrenzenden Fläche (240) liegt.

9. Sammelsystem (100) zum Anlegen an der peristomalen Haut, wobei das System Folgendes umfasst:
a. eine Wafer-Konstruktion (200) nach Anspruch 1-8; und
b. einen Sammelbehälter (300) zum Sammeln der Stomaausscheidung.

10. Sammelsystem (100) nach Anspruch 9, wobei das System einen löslichen Kleber (400) zum Auftragen auf der Haut vor dem Anlegen des Wafers umfasst.

11. Verfahren zum Bereitstellen einer Wafer-Konstruktion (200) oder eines Sammelsystems (100) nach einem der vorstehenden Ansprüche, wobei das Verfahren die Schritte umfasst zum:
a. Bereitstellen einer Wafer-Konstruktion (200) nach Anspruch 1-8; und
b. Packen der Wafer-Konstruktion.

## Revendications

1. Construction de plaquette (200) pour appliquer un système de collecte (100) autour d'une stomie, la construction de plaquette comprenant :
a. une ouverture centrale traversante (201) pour mettre la stomie (001) en communication fluidique avec un réservoir de collecte (300),
b. une couche interne s'étendant radialement (220) pour fixer la plaquette à la peau péri-stomiale (002) ; ladite couche interne (220) comprenant :
i. une surface de contact avec la peau (221) ;
ii. une couche perméable à la vapeur humide (224) ;
iii. une surface externe (226) ;
c. une couche externe s'étendant radialement (230) ; ladite couche externe comprenant :
i. un film sensiblement imperméable à la vapeur humide (232) ;
ii. une surface externe (236) ;
iii. une surface interne (238) ;
iv. un élément sac (234) pour mettre en prise la plaquette (200) avec un réservoir de collecte (300) ;
d. une zone centrale adjacente à la stomie (240) comprenant une première zone à la fois de la couche interne (220) et de la couche externe (230) ; ladite zone centrale adjacente à la stomie étant définie radialement en tant que zone de la construction de plaquette (200) plus près de l'ouverture centrale traversante (201) que l'élément sac (234) de la couche externe ;
e. une zone d'encerclement (250) comprenant une seconde zone d'au moins la couche interne (220) ;
f. au moins un espace radial (270) en communication fluidique avec l'atmosphère (500) ; ledit espace radial (270) étant situé entre la surface externe de la couche interne (226) et la surface interne de la couche externe (238) ; et
g. au moins un point de fixation (260) ; ledit point de fixation fixant la couche externe (230) à la couche interne (220) ;
dans laquelle au moins une partie de l'espace radial (270) est située dans la zone centrale adjacente à la stomie (240).

2. Construction de plaquette (200) selon la revendication 1, dans laquelle les points de fixation (260) sont situés à la fois dans la zone adjacente à la stomie (240) et dans la zone d'encerclement (250).

3. Construction de plaquette (200) selon la revendication 1 ou 2, dans laquelle le point de fixation dans la zone adjacente à la stomie est agencé en tant que jonction imperméable entre les couches interne et externe s'étendant radialement, ladite jonction imperméable s'étendant entièrement autour d'une stomie.

4. Construction de plaquette (200) selon la revendication 1, dans laquelle l'au moins un espace radial (270) comprend un moyen de séparation (271) ; ledit moyen de séparation facilitant la séparation de la couche interne s'étendant radialement (220) et de la couche externe s'étendant radialement (230) dans l'espace radial (270).

5. Construction de plaquette (200) selon l'une quelconque des revendications précédentes, dans laquelle la zone centrale adjacente à la stomie (240) comprend une partie de contact avec la stomie (245), ladite partie de contact avec la stomie étant adaptée pour entrer en contact avec la stomie.

6. Construction de plaquette (200) selon la revendication 5, dans laquelle la partie de contact avec la stomie (245) comprend un gel adhésif avec une dureté Shore A inférieure à 16 ; et ledit gel adhésif présentant une épaisseur d'au moins 1 mm.

7. Construction de plaquette (200) selon l'une quelconque des revendications précédentes, dans laquelle la surface de contact avec la peau (221) de la couche interne (220) comprend un adhésif cutané.

8. Construction de plaquette (200) selon l'une quelconque des revendications précédentes, dans laquelle le film sensiblement imperméable à la vapeur humide (232) de la couche externe s'étendant radialement (230) est situé seulement dans la zone centrale adjacente à la stomie (240).

9. Système de collecte (100) pour une application sur la peau péri-stomiale, ledit système comprenant :
a. une construction de plaquette (200) selon les revendications 1 à 8 ; et
b. un réservoir de collecte (300) pour collecter la sortie stomiale.

10. Système de collecte (100) selon la revendication 9, dans lequel le système comprend un adhésif soluble (400) pour une application sur la peau avant une application de la plaquette.

11. Procédé de fourniture d'une construction de plaquette (200) ou d'un système de collecte (100) selon l'une quelconque des revendications précédentes, ledit procédé comprenant les étapes consistant à :
a. fournir une construction de plaquette (200) selon les revendications 1 à 8 ; et
b. emballer la construction de plaquette.
